(19) Europäisches Patentamt
European Patent Office
Office européen des brevets

(11) **EP 2 149 789 A1**

(12) **EUROPEAN PATENT APPLICATION**

(43) Date of publication:
**03.02.2010 Bulletin 2010/05**

(51) Int Cl.:
**G01N 27/07** (2006.01)       **G01N 27/10** (2006.01)
**G01N 33/44** (2006.01)       **B29C 35/02** (2006.01)

(21) Application number: **09386020.3**

(22) Date of filing: **28.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **30.07.2008  GR 20080100507**

(71) Applicants:
• **Pantelelis, Nikolaos**
  **16122 Kaisariani (GR)**

• **Bistekos, Efthymios**
  **18541 Pireaus (GR)**

(72) Inventors:
• **Pantelelis, Nikolaos**
  **16122 Kaisariani (GR)**
• **Bistekos, Efthymios**
  **18541 Pireaus (GR)**

(54) **System for monitoring the moulding of reactive compounds like resins**

(57)    The patent is a complete system for monitoring the moulding process of reactive compounds and comprise one type of sensor (inline sensor) which is placed inline at the feeding or evacuation lines to/ from a mould, an electronic measuring system which applies constant voltage to the two electrodes of the sensor, being in contact with the monitored compound, and measures the current that runs through the circuit compound-electrodes-measuring system as well as, suitable algorithm that converts the measured current to information about the state of the compound (mixing quality, compound components, viscosity and/or degree of polymerisation) taking into consideration the simultaneous temperature measurement of the said compound.

The sensors comprise two electrodes in constant and very small distance between them while the electrical properties of the compound are measured as this flows or remains still inside the inline sensor. The system above may be part of a network of similar systems for the monitoring of the moulding of the compound in different locations of the production system such as the feeding lines, the evacuation lines, at the inlet or outlet gates and in the moulding cavity with the intention to acquire a complete overview of the progress of the reaction in the whole product. Using the system above or the network of systems it is possible to establish the remaining filling time of the cavity, the required temperature of the mould for the optimal cycle time as well as the quality certification of the end products.

Figure 2

## Description

### Context of the invention

The patent refers to a sensor, a system and a non intrusive method for monitoring the process of moulding reactive compounds based on the real-time measuring of the electrical resistance and temperature at appropriately selected locations. The patent comprise one basic shape of sensor which is installed in line with the feeding or the evacuation of the moulding compound and incorporates two electrodes which are kept in very small distance between them via an electrical insulator and through which the compound under investigation flows, a direct current measuring system which through suitable cables applies steady voltage at the electrodes of the sensor and measures the current which flows through the mass of the compound and depends from the state and the degree of reaction of this compound, as well as suitable algorithms for the analysis, the control, the recording and the depiction of the measurements using a computer. Application examples of the said system is the in-situ monitoring of the polymerisation of all types of thermoset resins such as polyesters, vinylesters, epoxies, polyurethanes, phenolics; reactive thermoplastics and others, with or without reinforcements such as glass fibres, carbon fibres and with or without additives. Using the system we can monitor the full production cycle of moulding the abovementioned compounds namely from the liquid state of the compound till the complete solidification without any intervention to this cycle. The inclusion of a temperature sensor in this sensor close to the electric sensing element provides through software, the potential of correlating the electrical signal with the physical and the chemical state (viscosity, chemical composition, degree of reaction etc.) of the compound that is in contact with the sensing element. Because of the instant measurement of the electrical conductivity we can also establish the state of a moving compound during its flow in a cavity or in any tube or gate in order to verify whether the reactive compound falls in the specs or not. The combination of multiple sensors in several appropriate locations either at the compound transfer lines to and from the mould cavity, or at the feeding and evacuation gates of the cavity or in the mould cavity itself can provide a more complete overview of the state of the compound and, finally, of the product.

### State of the Art

The difficulty of measuring some specific material properties during a production process in an industrial environment which are directly linked to the product quality such as the chemical composition, the viscosity and the degree of reaction led to correlate these properties with others such as the electrical properties which can be easier measured without any burden in the production cycle. Furthermore it is well-known that the measurement of the materials dielectric constant can provide significant information for their structure as well as their physical (composition, homogeneity, melting, solidification etc.) and chemical (polymerisation etc.) processes that perhaps are taking place in the materials. The measurement of the materials dielectric properties is based on the application of alternative current or voltage at the sensing element and measuring its feedback, and appropriate systems and sensors are commercially available whereas numerous patents exist e.g. Magill et al, Patent US 7,245,985: "Process and apparatus for improving and controlling the vulcanization of natural and synthetic rubber compounds" and scientific papers e.g. D. Kranbuehl, S. Delos, E. Yi, J. Mayer, T. Jarvie, W. Winfree, T. Hou, "Dynamic Dielectric Analysis: Nondestructive Material Evaluation and Cure Cycle Monitoring", Polymer Engineering and Science, 1986, Vol. 26(5), pp. 338-345. On the contrary, the measurement of the materials' electrical conductivity using high DC voltage (over 100 V) is used mostly for the characterization of insulating materials by measuring the specific volume resistance (volume resistivity) and the specific surface resistance (surface resistivity) based on the ASTM D-257 as well as in geology. However, neither the application of direct voltage for the measurement of the electrical properties in materials moulding or processing nor sensors similar to the present sensor have been used systematically.

At research level, measuring the electrical resistance of liquid state resins where the resistance level is quite low has been measured with simple bridge-type direct current equipment and simple sensors e.g. Tajima, "Monitoring Cure Viscosity of Epoxy Composite", Polymer Composites, Vol. 3, No. 3, pp. 162-169. One of the first who used direct current (DC) in monitoring the moulding of composite materials was Walsh who in the patent US 5,210,499: "In-situ method sensor and device", proposed a grid of cables which create a network of point measurements through stripping and insulating parallel and transverse cables and the application of direct current at their ends. Basic differences with the present system is that the Walsh sensor has a completely different shape, lower sensitivity and larger size and remains in the final product while a different connectivity for the measurement of the current that runs through the compound when direct voltage is applied to the electrodes of the sensor was used and furthermore, high resistance measurements is not possible. In the same philosophy is the sensor that proposed by King, West and Bernardon, US 5,528,155: "Sensor for measuring material properties" in which cables were also used as the basic sensor type together with the same measuring bridge circuit.

Another relevant patent that relates to the monitoring a production process is the patent of M.McBrearty and S.Perusich, US 5,208,544: "Noninvasive dielectric sensor and technique for measuring polymer properties", in which the sensor is placed at the injection head of polymer melt, however the sensing element has an interdigitate shape, the measuring technique is based on the application of alternative current whereas the application is quite different for which the patent was developed for.

A measuring technique that uses a sensor of similar shape with the present invention is presented by D.S.Goldman and S.Wilcox, US 5,612,622: "Apparatus for identifying particular entities in a liquid using electrical conductivity characteristics", in which the sensor is placed externally to a feeding line so no physical contact with the measured liquid exists, also alternative current is applied to the two electrodes of the sensor whereas completely different is the application for which the patent has been developed for.

Finally, in an attempt to isolate the electrode polarization due to the applied electrical field the application of a characteristic time varying voltage has been proposed by Boiteux et al, EP 0733902B1: "Procede et dispositive de suivi de polymerisation des systemes reactifs".

In the modem industrial production, during the moulding of reactive compounds with or without reinforcement the only physical property that is being measured and taken under consideration besides time is the temperature of the mould or the compound. However, it is obvious that just the temperature cannot provide any direct information for the real state of the materials in a mould so this can not be used for real quality control or process control purposes. In other words the patented system provides the user with the ability to supervise the production and consequently with the potential for quality control of the products without any substantial intervention to the manufacturing process or to the product itself.

**Presentation of the invention**

The invention comprises a main sensor type (inline sensor) while it uses also a subsidiary sensor type (cavity sensor) of different shape and use. The two types of sensor are based on the measurement of the same physical phenomenon, the electrical resistance and the temperature of the material under investigation. The sensors can operate in constant contact and material pressure between -1 and +20 bar and in temperatures up to 350°C while they show good durability so they can be used for numerous production cycles without hindering the production process or to unfavorable affect the product.

The inline sensor of the invention has a through hole of cylindrical or conical shape so the compound can flow through it and is based on the existence of two electrodes in very small distance. The shape of the electrodes and the auxiliary components of the inline sensor are such that they do not obstruct the flow of the measured compound when this flows through its body. The inline sensor has two types: the "inline external sensor" and the "inline gate sensor".

The inline gate sensor is placed either at the feeding gate of the cavity or at the evacuation gate inside or in a small distance from the mould in such a way that allows the direct contact of the compound with the electrodes of the sensor. It can be also placed at the margin of the main moulding cavity (technical area) which is formed between the upper and lower parts of the mould when these are clamped and the peripheral sealing of the mould cavity. The compound inside the sensor is at similar temperature with the compound in the cavity so they are also at similar physical and chemical states. By applying a constant voltage to the two electrodes of the sensor reliable electrical measurements are taken even if the reactive compound flows through the sensor. The inline gate sensor has a conical shape so that it enables the easy removal of the solidified compound from inside the sensor so it can be reusable. For this reason the inline gate sensor can be also used for measuring completely solidified compounds. The most significant advantages of the gate sensor are the capability of monitoring the state of the compound that has flown in the moulding cavity without the need of an additional hole in the mould as well as the measurement of the neat compound before this enters the moulding cavity and becomes polluted from substances and materials that are already in the moulding cavity such as release agents, cleaning agents, binders, reinforcements such as glass, carbon or other polymer fibres, polymer foams etc. Even though these materials do not affect particularly the main chemical and physical properties of the compound they may affect significantly and randomly the electrical resistance of the compound in the cavity.

The inline external sensor is placed in line either in one or in every feeding or the evacuation line of the cavity in such a way the compound can flow through the sensor without obstructing the flow. Its two ends are appropriately formed so that the ends of the feeding lines can be attached. With the application of constant voltage in the electrodes of the inline external sensor it is possible to directly measure the electrical properties of the flowing or still compound in it. It possesses a mechanically identical to the inline gate sensor internal part for measuring device but differs in the external design. The most significant advantage of the inline external sensor when it is placed in a feeding line is the establishment of the physical state of the compound e.g. viscosity or its chemical state e.g. composition or degree of reaction, as this is transferred from the injection machine to the mould cavity. This sensor should be either cleaned with the feeding line otherwise the injection of the compound should start before the existing compound starts to solidify according to the same practice that is being followed in the industry for the lines of the injection systems.

The sensor system for the monitoring of the moulding process includes also a measuring device which applies constant voltage to the two electrodes and measures directly the electric current that runs the circuit measuring device- electrodes which is closed when the compound is in contact with the electrodes of the sensor. The analog electrical signal is digitized and transferred to a computer for further processing and storage. So these electrical measurements can be converted to resistance units and can be depicted in appropriate diagrams.

At this point the significance of some geometrical and electrical characteristics for the achievement of the targets of the invention should be clarified. Firstly, most of the resin compounds for composite materials present very high electrical resistance levels. So when these are in liquid state they present a surface resistivity higher than $10^8$ Ω whereas when these are completely solid the surface resistivity can reach $10^{14}$ Ω ($10^6$ increase).

Lower but equally significant effect to the surface resistivity has the temperature of the compound. The in situ measurement of such high values is a challenge because of the high noise levels of an industrial environment and the geometrical limitations to the sensor size. Based on the theory (ASTM D 257-07, «Standard Test Methods for DC Resistance or Conductance of Insulating Materials») the surface resistivity of a material is given as:

$$\text{surface electrical resistance} = (\text{surface resistivity}) * (\text{electrodes' gap})/ (\text{mean value of the two electrodes' perimeters})$$

So for a given surface resistivity of a specific compound and temperature, the measured electrical resistance decreases when the distance between the electrodes is decreased and the electrodes' active perimeter is increased. So for the successful measurement of the electrical resistance of the compound during the whole range of the production process a geometric optimization of the corresponding sensor is required so that the electrical resistance range of the compound falls into the measuring device range. For a typical sensor size (active electrodes' perimeter in the order of $10^{-2}$ m) the minimum distance between the electrodes should not be lower than $10^{-5}$ m as the probability of compound debris between the electrodes is highly increased resulting in faulty measurement in the next measurement. If however the distance between the electrodes becomes exceeds $3*10^{-4}$ m the measurement becomes extremely weak resulting in difficulty of the processing of the measurements and reduced reliability due to parasitic voltages especially during the compound's solidification.

For the calculation of the electrical resistance Ohm's law is applied:

$$\text{electrical resistance} = (\text{voltage}) / (\text{measuring current})$$

This law is applied in suitable electronic circuit which applies constant voltage to the electrodes of the sensor, which coming in contact with the compound closes the circuit is so the current under measurement is created. This current increase with the increase of the voltage applied to the circuit. As an upper voltage limit the 24 volts have been selected to avoid electroshocks and to reduce the electric fields generated at the sensor's cabling. As a lower limit the 0.1 volts were selected under which polarization phenomena affect significantly the measurements making them unreliable. Measuring simultaneously the temperature and the electrical resistance of a reactive compound we have all the data for calculating with suitable algorithms the degree of reaction of the compound, the viscosity, the composition, the aging and any other characteristic that can be correlated to the combination of temperature and electrical resistance of the compound. For the execution of the above calculations the measuring device can be used with any of the said sensors.

The said system constitutes the means with which we are capable to monitor in real-time the moulding of reactive compounds at the selected measuring location. We can also employ one or more such systems, to place the corresponding sensors at suitable locations while the reception, the processing and the analysis of all these measurements can provide directly significant information for the complete state of the process without any intervention either to the production process or to the product. Thus after suitable calculations it is possible to establish:

➢ The remaining time for keeping the compound in the feeding line before purging and cleaning is necessary

➢ The arrival of the compound at the evacuation or vacuum lines

➢ The quality of the mixing and the mixing ratio of the ingredients of the compound in real time

➢ The progress of the moulding

➢ The required time to complete the reaction of the compound

➢ The achievement of important milestones in the reaction of the compound

➢ The required mould temperature so that the required reaction time is constant for each cycle.

The above indicative information gives us the capability through recording to certify the stability of the quality during the composite material production and as a result making the said system essential of the quality certification of the final products.

**Detailed description of the invention**

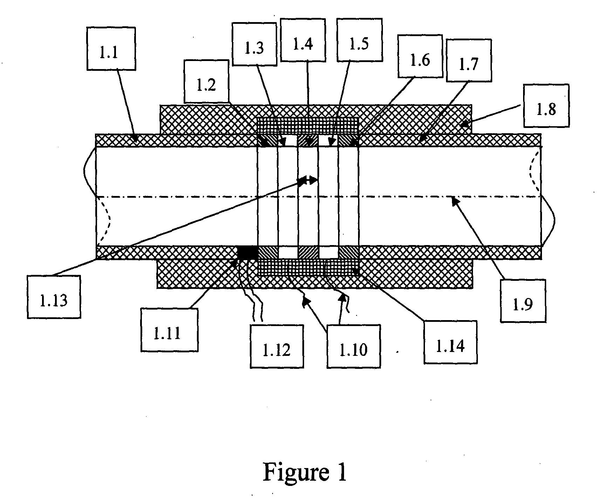

Figure 1 illustrates a longitudinal cross section of an application example of the inline sensor which is positioned in the feeding line of the compound to the mould through elements (1.1) and (1.7) and comprise two pierced cylindrical electrodes (1.3) and (1.5) which are maintained at constant distance having in between another cylinder (1.4) made of dielectric material which, due to manufacturing purposes, may be integrated to one or both electrodes. The height of the pierced cylindrical electrodes (1.3) and (1.5) can be from 50 $\mu$m up to 10 mm while the height (1.13) of the pierced insulating cylinder (1.4) which has the shape of a thin ring, must be between 10 $\mu$m and 300 $\mu$m. Additional pierced cylinders made from insulation material are placed on both sides (1.2, 1.6) and externally (1.14) to the electrodes (1.3, 1.5) in such a way that these are electrically insulated from the outer sheath, while the flow of the compound takes place through the cavity (1.9) existing in the sensor. The selection of the location of the temperature sensor (1.11) near its electrodes is based on three criteria, first the high thermal conductivity, second the manufacturability and third the cables' arrangement. All of the above components are binded by a sheath (1.8) through which the cables that connect the electrodes (1.10) and the temperature sensor (1.12) to the measuring system pass across.

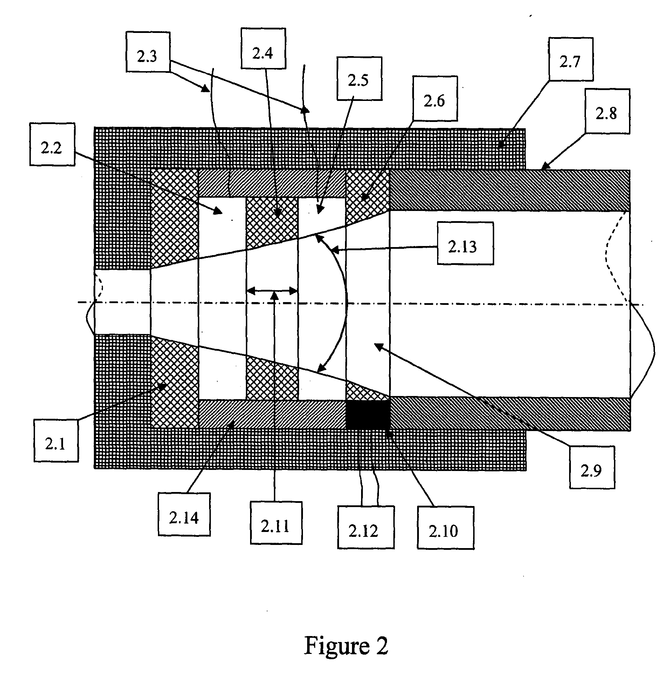

In figure 2 an example of an inline gate sensor is presented which as depicted in its longitudinal cross section, comprises two electrodes (2.2) and (2.5) which are kept in constant distance between them with a pierced cylinder (2.4) from dielectric material which is used for the electrical insulation of the electrodes. The conical cavity through which the compound flows has a tapering angle (2.13) between 0° and 30° whereas its tapering direction depends on the possibility of cleaning the cavity and not on the flow direction of the compound. The height of the insulating cylinder (2.11) is between 10 $\mu$m and 300 $\mu$m. At both sides of the two electrodes (2.2) and (2.5) two additional pierced cylinders (2.1) and (2.6) are placed which are used for the electrical insulation of the electrodes. The cylinders (2.1) and (2.6) have similar tapering angles as the cylinders (2.2) and (2.5). All of the said cylinders are positioned as depicted in figure 2 in such a way that an internal conical cavity (2.9) is formed from which the solidified compound can be easily removed after the reaction of its ingredients. For improved accuracy of the calculation of the state of the compound its temperature should be also recorded using a temperature sensor (2.10) which is electrically insulated from the two electrodes (2.2) and (2.5). All of the above components are binded by a sheath (2.7) which is also used for the installation of the sensor in the mould and is in touch with the mould cavity allowing the electrodes' cables (2.3) and the temperature sensor cables (2.12) to safely come out towards the measuring system. For the electrical insulation of the electrodes from the sheath another pierced cylinder (2.14) made of dielectric material is placed between the cylinders (2.2), (2.4) and (2.5) and the sheath (2.7). The mechanical support of the sensor with the feeding or the evacuation of the mould is realised using the element (2.8).

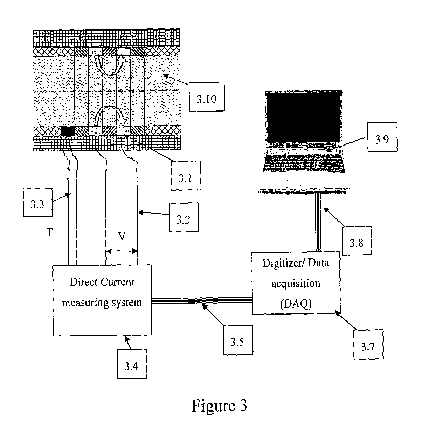

In figure 3 a typical diagram of a system for monitoring the moulding of reactive compounds is displayed where an inline sensor at the feeding line (3.1) is shown, through which the monitored compound (3.10) flows, at its electrodes constant voltage (3.2) is applied and the corresponding current is recorded through a suitable electronic measurement system (3.4). In the same measuring system the cables (3.3) from the temperature sensing element are coupled. The corresponding signals are transferred through line (3.5) to the digitizer (3.7) where after suitable processing these signals are digitized and transferred through cables (3.8) they stored at the computer system (3.9).

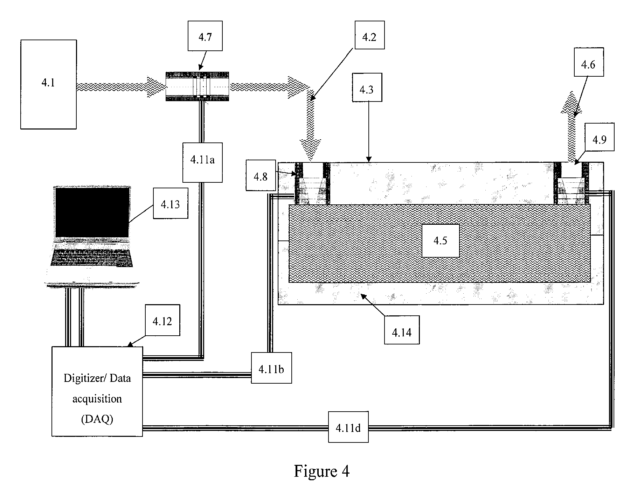

In figure 4 a schematic example of a complete monitoring system for moulding reactive compounds is depicted. The basic production system of a reactive compound monitoring system comprise a mixing and injection machine for the compound (4.1) which is piped through the feeding line (4.2) in the mould cavity (4.5) which is formed from the upper (4.3) and the lower (4.14) tools. The air and the overflow compound of the cavity are evacuated through the outlet gate (4.6). In the said production system an inline sensor is placed at the feeding line, an inline gate sensor (4.8) is placed at the inlet gate of the moulding cavity, an inline gate sensor (4.9) is placed at the outlet gate (4.6) of the compound from the moulding cavity. Each sensor is linked with a separate but similar measuring system (4.11a), (4.11b), (4.11c) which then are linked to a common digitizer/ data logger (4.12) which digitizes the measurements of the electrical current and temperature from each sensor which they are transferred to the computer system (4.13) for processing and storage.

**Advantages of the invention**

The main sensor type is placed in line either at the feeding line of the compound to the moulding cavity, either at the inlet gate of the compound to the mould, or at the inlet gate or even at the outlet gate of the mould. The verification of the required compound viscosity, the right quality of the mixing ratio of the compound ingredients and even the mixing quality of its ingredients is possible in real time as the sensor's shape is such that the running compound inwards or outwards of the mould cavity is not obstructed while the electrical resistance and the temperature can be measured simultaneously when the compound runs through or rests in the sensor. Also the inline sensor can be used after the end of the injection to monitor the progress of the reaction of the compound which resides in it and out of the cavity so either the progress of the reaction of the compound is possible (if the temperature difference between the mould cavity and the location of the sensor is small otherwise using calculations and statistics) or the avoidance of flushing of the feeding lines in recurring production cycles.

Especially in the former case the inline gate sensor can provide important information on the degree of reaction of the compound in the cavity reflecting the state of the most "fresh" (because of the most recent mixing) compound so the system can provide the user with the indication of the degree of reaction of the least polymerized mass compound in the cavity so consequently if it is shown that the degree of reaction in that location has reached the desired level then we are sure that at least the same has happened for the totality of the compound in the cavity. It should be highlighted that this valuable information is obtained without any intervention to the mould or the moulding process. The inline sensor (external or gate) when placed at the feeding line of the cavity provides the important advantage of sensing the pure compound from clear from any impurities or fibers that may exist in the moulding cavity especially when reinforced material is pre-placed in the cavity.

If the inline (external or gate) sensor is placed in the compound evacuation line from the moulding cavity it can provide additional information of the state of the compound after it has flown considerable distance in the moulding cavity so we can record the exact time of the resin overflow from the cavity as well as the degree of reaction of the compound at this point so e.g. excessive progress of the reaction of the compound can be identified.

Finally, in the case of multiple feeding lines, when the inline gate sensor is placed in another inlet gate it is possible to trace the arrival of the compound at this specific gate and thus to automate the start of the injection from this specific gate.

Furthermore in the case that more than one sensor exist also internally in the cavity or at the outlet gates the correlation of the measurements from all the sensors simultaneously is possible so a more representative measurement of the degree of reaction of the totality of the compound and consequently a reliable quality control of the production process can be achieved.

Based on the monitoring method of the moulding process it is possible after appropriate calculations to establish the remaining filling time of the cavity with the reactive compound, the required time for concluding the reaction of the compound, the necessary temperature of the mould so for each cycle the required time for concluding the reaction of the compound is constant and other features of the production that may be useful for the improvement of the production process.

EXAMPLES

EXAMPLE 1. ESTABLISHMENT OF MIXING RATIO VARIATION IN A EPOXY RESIN/ HARDENER COMPOUND

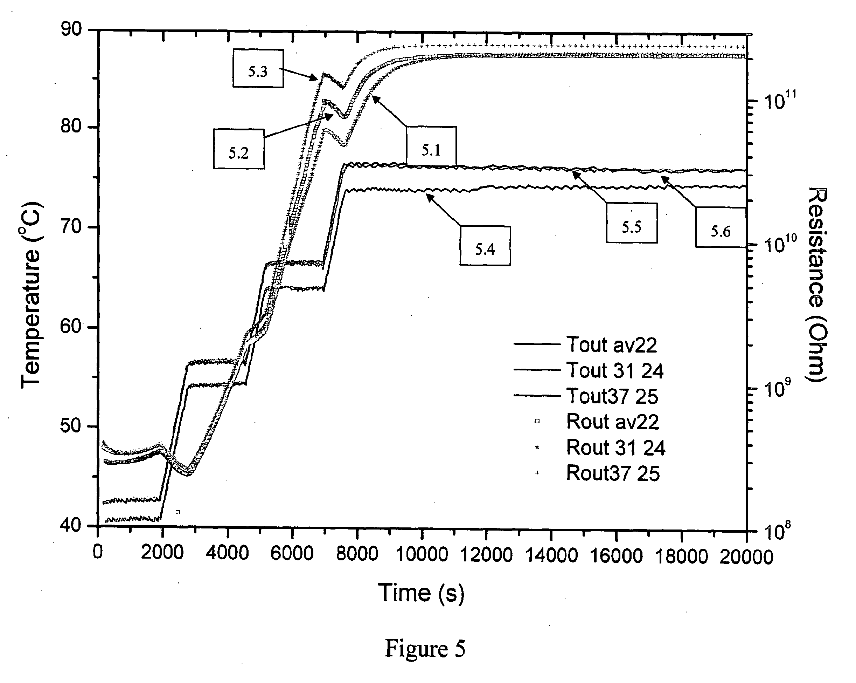

An inline gate sensor was manufactured having mean internal diameter of 10 mm at the location of the electrodes which are kept apart at a distance of 0.20 mm. The sensor was placed in line with a feeding line close to the central gate of the mould at an experimental set-up and mixed epoxy resin L20 with EPH960 hardener, both products of Bakelite, were injected in three different experiments and mixing ratios of 100:31 (resin mass/ hardener mass), 100:34 and 100:37. Using the inline gate sensor and the corresponding measuring system the variations of electrical resistance of the compound together with its corresponding temperature were recorded as depicted in figure 5. So curve (5.1), showing the change of the resistance during the reaction of the aforementioned compound at the imposed temperature profile of curve (5.5) corresponds to a lower ratio of hardener (31 gr of hardener for 100 gr of resin). Respectively, curve (5.2) showing the variation of the electrical resistance of this compound at the imposed temperature profile of curve (5.4) corresponds to the nominal mixing ratio (34 gr of hardener for 100 gr of resin) and finally curve (5.3) showing the variation of the resistance during the reaction of the aforementioned compound at the imposed temperature profile of curve (5.6) corresponds to a higher ratio of hardener (37 gr of hardener for 100 gr of resin). From the study of the above results can be deduced that the delay in the reaction of the compound with reduced or increased the hardener's percentage in the compound corresponds well with the delay/ acceleration, respectively, in the rise of the electrical resistance of the compound.

EXAMPLE 2. VERIFICATION OF AGED PREMIXED COMPOUND OF EPOXY RESIN/ HARDENER

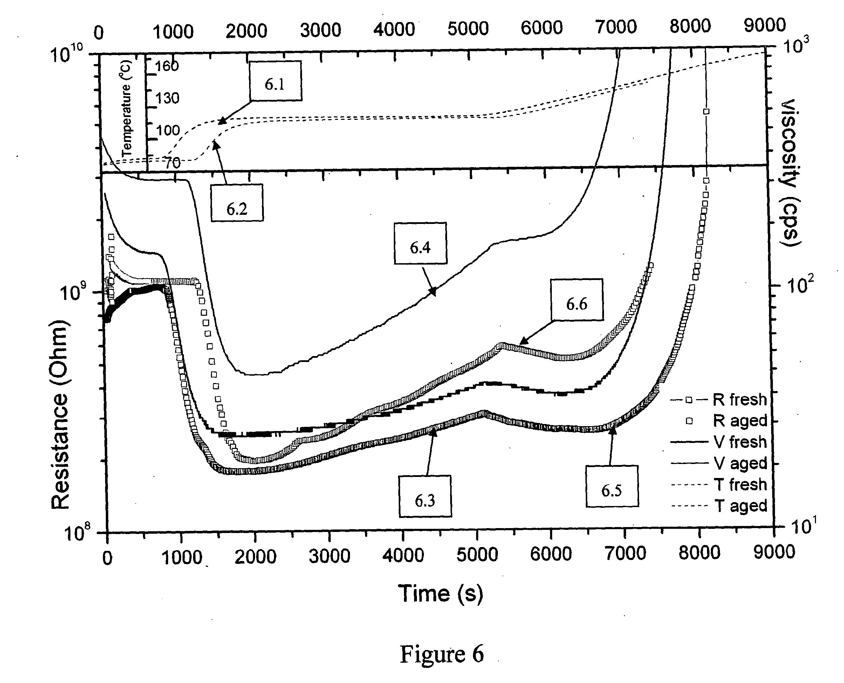

An inline gate sensor was manufactured having mean internal diameter of 5 mm at the location of the electrodes which are kept apart at a distance of 0.080 mm. The sensor was placed at a special set-up in a viscometer in such a way that simultaneous measurements of viscosity, electrical resistance and temperature of the reactive compound were obtained. In this set-up two different experiments were executed, one with fresh and one with aged premixed epoxy system (RTM6 of Hexcel Composites). The obtained electrical measurements are noted in figure 6 as electrical resistance. In the same figure 6 the temperature profile of the normal resin (6.1), the corresponding viscosity curve (6.3) and the corresponding electrical resistance of the fresh resin (6.5) are shown. Also in figure 6 the temperature profile of the aged resin (6.2), the corresponding viscosity curve (6.4) and the corresponding electrical resistance of the aged resin (6.6) are shown. As expected, the aged resin shows increased viscosity but also faster viscosity rise (comparing curves (6.3) and (6.4)) with respect to the fresh resin, a fact that confirms the corresponding values and rates of the respective electrical resistance curves (comparing curves (6.5) and (6.6)).

EXAMPLE 3. MONITORING VISCOSITY CHANGES OF AN EPOXY RESIN

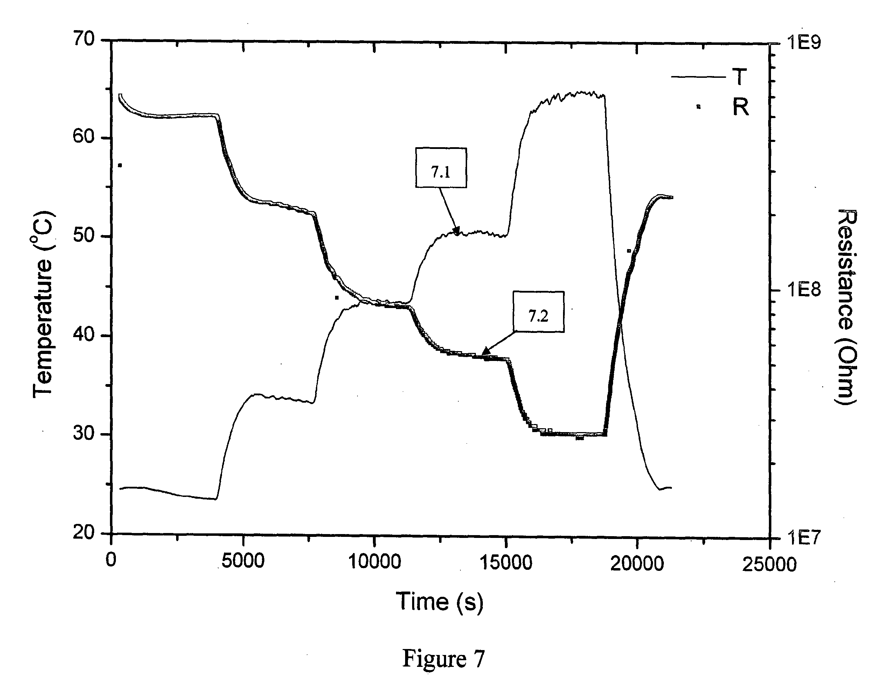

An inline gate sensor was manufactured having mean internal diameter of 10 mm at the location of the electrodes which are kept apart at a distance of 0.20 mm. The sensor was placed in line with a feeding line close to the central inlet gate of a heated mould. In this set-up, an experiment of monitoring the physical state of epoxy resin L20 of Bakelite without hardener during the temperature rise of the housing of the sensor. As depicted in figure 7 imposing the temperature profile (7.1) as measured by the temperature sensor of the inline external sensor, the electrical measurements of the resin denoted as electrical resistance (7.2) follow exactly the temperature changes of the resin as expected, namely as the temperature of the resin is rising, its viscosity drop is represented characteristically with the drop of its electrical resistance.

**Claims**

1. Sensor for the in-situ monitoring of the moulding process of reactive compounds which is **characterised by** a through hole, through which the reactive compound can flow and its inner measuring surface is formed by two pierced cylindrical electrodes electrically insulated (between them and from the sheath of the sensor with high electrical resistivity materials), across and coaxially placed, in direct contact with the compound and to which constant voltage is applied, the current that runs the electrodes is recorded together with the temperature of the compound so that with the aid of appropriate algorithm the physical and chemical state of the compound can be calculated.

2. Sensor of claim 1 which is connected in line in any location of the feeding or evacuation lines of the moulding cavity including the feeding or evacuation gates of the moulding cavity.

3. Sensor of claim 1, the inner through cavity of which is conical so when the sensor is placed in any of the existing gates of the moulding cavity the easy release of the solidified compound is possible.

4. Sensor of claim 1, the electrodes (1.3 and 1.5, 2.2 and 2.5) of which are at the contact surface with the monitored compound and are kept in constant distance between them (1.13, 2.11) from 10 to 300 $\mu$m.

5. Sensor of claim 1, the electrodes of which are isolated between them and from the sheath of the sensor using dielectric materials.

6. Sensor of claim 1 at the electrodes of which constant voltage from 0.1 to 24 Volts is applied.

7. System for the in-situ monitoring of the moulding of reactive compounds with or without reinforcement comprising

   a sensor of claim 1 which is placed in line with the feeding or evacuation line in such a way that its electrodes are in constant contact with the compound electronic system which applies constant voltage at the sensor's electrodes and measures the current that runs the circuit
   temperature sensor element of the monitored compound
   algorithm which using the said current and temperature measurements calculates in real-time the viscosity, the chemical composition and the degree of reaction of the compound

8. Electronic system of claim 7 which by applying constant voltage at the electrodes of the sensor of claim 1 measures in real-time the current that runs the circuit which is formed by the mass of the reactive compound which is in contact with the said electrodes and may be moving.

9. Method for the complete monitoring of the moulding of reactive compounds with or without reinforcement in which

one or more sensors of claim 1 are placed in feeding and evacuation lines in contact with the compound
constant voltage is applied to the electrodes of each said sensor and the generated current is measured
from the measured current and temperature of the compound the state of the compound that is in contact with
each of the said sensors is defined individually at this instance and
from the local states of the compound in the cavity, at the feeding and the evacuation lines the complete picture
of the state of the moulded compound is established through calculations.

Figure 1

Figure 2

Figure 3

Figure 4

Figure 5

EP 2 149 789 A1

Figure 6

Figure 7

EP 2 149 789 A1

| | Europäisches Patentamt European Patent Office Office européen des brevets | **EUROPEAN SEARCH REPORT** | Application Number EP 09 38 6020 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 60 206836 A (KOGYO GIJUTSUIN) 18 October 1985 (1985-10-18) * abstract; figures 1-3 * ----- | 1-9 | INV. G01N27/07 G01N27/10 G01N33/44 |
| A | NIKOS PANTELELIS: "Composite material process monitoring using durable sensors" AEROSPACE 08, [Online] 15 April 2008 (2008-04-15), XP002533004 Munich, Germany Retrieved from the Internet: URL:http://www.aerospacetesting.com/files/pantelelis_Aerospace08.pdf> [retrieved on 2009-06-17] * the whole document * ----- | 1-9 | B29C35/02 |
| A | SCHWAB SCOTT D ET AL: "Sensor system for monitoring impregnation and cure during resin transfer molding" POLYMER COMPOSITES 1996 APR SOC OF PLASTICS ENGINEERS, vol. 17, no. 2, April 1996 (1996-04), pages 312-316, XP002533005 * abstract; figures 1-7 * * chapter "EXPERIMENTAL" * ----- | 1-9 | |
| Y | DD 126 416 A1 (HILDEBRAND, KONRAD) 13 July 1977 (1977-07-13) * paragraph bridging pages 3,4 * * abstract; figure 1 * ----- | 1-9 | TECHNICAL FIELDS SEARCHED (IPC) G01N B29C |
| A | DE 22 43 915 A1 (MALCOM ELLIS LIVERPOOL) 14 March 1974 (1974-03-14) * figures 1,2 * ----- | 1-9 | |
| A | DE 26 17 007 A1 (M E MEERESTECHNIK ELEKTONIK GM) 27 October 1977 (1977-10-27) * figure 1 * ----- | 1-9 | |

The present search report has been drawn up for all claims

| Place of search | Date of completion of the search | Examiner |
|---|---|---|
| Munich | 19 November 2009 | Strohmayer, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 38 6020

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2009

| Patent document cited in search report | | Publication date | Patent family member(s) | | Publication date |
|---|---|---|---|---|---|
| JP 60206836 | A | 18-10-1985 | JP<br>JP | 1813557 C<br>5025891 B | 18-01-1994<br>14-04-1993 |
| DD 126416 | A1 | 13-07-1977 | CS<br>SU | 204150 B1<br>667880 A1 | 31-03-1981<br>15-06-1979 |
| DE 2243915 | A1 | 14-03-1974 | NONE | | |
| DE 2617007 | A1 | 27-10-1977 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7245985 B, Magill
- US 5210499 A
- US 5528155 A, King, West and Bernardon
- US 5208544 A, M.McBrearty and S.Perusich
- US 5612622 A, D.S.Goldman and S.Wilcox
- EP 0733902 B1, Boiteux

### Non-patent literature cited in the description

- **D. Kranbuehl ; S. Delos ; E. Yi ; J. Mayer ; T. Jarvie ; W. Winfree ; T. Hou.** Dynamic Dielectric Analysis: Nondestructive Material Evaluation and Cure Cycle Monitoring. *Polymer Engineering and Science,* 1986, vol. 26 (5), 338-345
- **Tajima.** Monitoring Cure Viscosity of Epoxy Composite. *Polymer Composites,* vol. 3 (3), 162-169